# EUROPÄISCHE PATENTSCHRIFT

(11) **EP 3 247 695 B1**
(45) Veröffentlichungstag und Bekanntmachung des Hinweises auf die Patenterteilung: **06.03.2019**
(21) Anmeldenummer: 16700972.9
(22) Anmeldetag: 18.01.2016
(51) Int. Cl.: C07C 51/31, C07C 51/48, C07C 53/02

(54) **VERFAHREN ZUM ABSONDERN VON AMEISENSÄURE AUS EINEM REAKTIONSGEMISCH**
METHOD FOR ISOLATING OF FORMIC ACID FROM A REACTION MIXTURE
PROCÉDÉ DESTINÉ À EXTRAIRE UN ACIDE FORMIQUE D'UN MÉLANGE RÉACTIF

(30) Priorität: 19.01.2015 EP 15151654
(43) Veröffentlichungstag der Anmeldung: 29.11.2017
(73) Patentinhaber: OxFA GmbH, 96110 Scheßlitz (DE)
(72) Erfinder: ALBERT, Jakob, 91080 Marloffstein (DE); BÖSMANN, Andreas, 91093 Heßdorf (DE); REICHERT, M. Sc. Jenny, 97523 Schwanfeld (DE); WASSERSCHEID, Peter, 91054 Erlangen (DE)
(74) Vertreter: Dr. Gassner & Partner mbB
(86) Internationale Anmeldenummer: PCT/EP2016/050903
(87) Internationale Veröffentlichungsnummer: WO 2016/116405

(56) Entgegenhaltungen:
- EP-B1- 1 230 206
- EP-B1- 2 473 467
- WO-A1-2009/130386
- WO-A1-2013/078391

## Beschreibung

Die Erfindung betrifft ein Verfahren zum Absondern von Ameisensäure aus einem Reaktionsgemisch, welches neben der Ameisensäure ein Polyoxometallat-Ion umfasst.

Ein solches Verfahren ist aus der EP 2 473 467 B1 bekannt. Dabei kann die Ameisensäure mittels Extraktion abgesondert werden. Zur Extraktion kann ein Ether oder ein Amid eingesetzt werden.

Aus der EP 1 230 206 B1 ist ein Verfahren zur Herstellung von Ameisensäure bekannt. Dabei wird Methylformiat unter Erhalt eines Gemischs aus Wasser, Ameisensäure, Methanol und überschüssigem Methylformiat hydrolysiert und das Methanol und das überschüssige Methylformiat unter Erhalt von wässriger Ameisensäure destillativ abgetrennt. Die wässrige Ameisensäure wird dann mit mindestens einem Ameisensäureester aus der Gruppe, die gebildet ist von Ethylenglycoldiformiat, Diethylenglycoldiformiat, 1,2-Propandiodiformiat, 2,3-Propandioldiformiat, Dipropylenglycoldiformiat, 2,3-Butandioldiformiat, 1,4-Butandioldiformiat, Benzylformiat, Cyclohexylformiat, 2-Phenylformiat und 2-Ethylhexylformiat unter Erhalt eines Gemischs aus Ameisensäureester und Ameisensäure unter Destillationsbedingungen in einer Extraktivdestillationskolonne extrahiert. Das erhaltene Gemisch aus Ameisensäureester und Ameisensäure wird anschließend destillativ getrennt. Das Verfahren ist verhältnismäßig aufwendig, da es eine Extraktivdestillationskolonne und mehrere Destillationsschritte erfordert.

Aus der WO 2009/130386 A1 ist ein Verfahren zur Gewinnung von Ameisensäure bekannt. In einem Ausführungsbeispiel wird Ameisensäure aus einer sauren Hydrolyse von Biomasse mittels Octanol aus einer wässrigen Mischung extrahiert.

Die WO 2013/078391 A1 offenbart ein Verfahren zur Herstellung von Lävulinsäure, Ameisensäure und/oder Hydroxymethylfurfural aus verschiedenen Biomassen.

In den Ausführungsbeispielen wurden verschiedene organische Lösungsmittel, wie beispielsweise n-Hexanol, n-Heptanol, n-Octanol, 1-Nonanol und 1-Undecanol, zur Extraktion von Lävulinsäure, Ameisensäure und Schwefelsäure aus einer wässrigen Lösung verwendet.

Aus Khenkin, A. M. und Neumann, R., J. AM. CHEM. SOC. 2008, 130, 14474 bis 14476 ist eine oxidative Spaltung von C-C-Bindungen primärer Alkohole und vicinaler Diole, wie 1,2-Ethandiol, bekannt. Als Katalysator wird dabei H₅PV₂Mo₁₀O₄₀ verwendet. Die primären Alkohole wurden dabei in Dioxo-tetrahydrothiophen unter streng anaeroben Bedingungen umgesetzt. Als primäre Alkohole wurden u. a. lineare Alkohole mit 4, 5 und 6 Kohlenstoffatomen eingesetzt.

Aufgabe der vorliegenden Erfindung ist es, ein alternatives Verfahren zum Absondern von Ameisensäure aus einem Reaktionsgemisch, welches neben der Ameisensäure ein Polyoxometallat-Ion umfasst, anzugeben.

Die Aufgabe wird durch die Merkmale des Patentanspruchs 1 gelöst. Zweckmäßige Ausgestaltungen ergeben sich aus den Merkmalen der Patentansprüche 2 bis 9.

Erfindungsgemäß ist ein Verfahren zum Absondern von Ameisensäure aus einem Reaktionsgemisch vorgesehen, wobei das Reaktionsgemisch neben der Ameisensäure ein Polyoxometallat-Ion der allgemeinen Formel [PMoₓV_{y}O₄₀]ⁿ⁻ umfasst, wobei 6 ≤ x ≤ 11, 1 ≤ y ≤ 6, x + y = 12 und 3 < n < 10, wobei n, x und y jeweils eine ganze Zahl ist. Bei einer Ausgestaltung des Verfahrens ist n = 3 + y. Die Ladung kann in Abhängigkeit von den Bedingungen in dem Reaktionsgemisch, wie z. B. dem pH-Wert, auch andere Werte von 4 bis 9 annehmen. Das Absondern erfolgt durch eine Extraktion mittels eines linearen primären Alkohols, wobei die Kohlenstoffkette des Alkohols jeweils 5 bis 12 Kohlenstoffatome aufweist. Das Reaktionsgemisch liegt dabei in einem protischen Lösungsmittel vor.

Das Reaktionsgemisch kann beispielsweise ein Reaktionsgemisch aus einem Verfahren zur katalytischen Erzeugung von Ameisensäure sein. Dabei dient das genannte Polyoxometallat-Ion als Katalysator. Der Katalysator kann bei einer Temperatur unterhalb von 120°C mit einem alpha-Hydroxyaldehyd, einer alpha-Hydroxycarbonsäure, einem Kohlenhydrat oder einem Glycosid als Substrat in einer flüssigen Lösung in Kontakt gebracht werden, wie es in der EP 2 473 467 B1 beschrieben ist. Weiterhin kann das Reaktionsgemisch ein den Aufschluss des Substrats förderndes Additiv, wie z. B. Toluolsulfonsäure, enthalten. Bei der Umsetzung des Substrats mit dem genannten Katalysator entsteht neben Ameisensäure in unterschiedlichem Ausmaß CO₂. Das erfindungsgemäße Verfahren ermöglicht die Extraktion der Ameisensäure aus diesem Reaktionsgemisch während der Reaktion zur Erzeugung der Ameisensäure unter für die Reaktion günstigen Reaktionsbedingungen, d. h. zum Beispiel bei einer Temperatur von 50°C bis 120°C, insbesondere 60°C bis 100°C, insbesondere 70°C bis 95°C, insbesondere 85°C bis 95°C. Es ermöglicht die Extraktion der Ameisensäure aus dem Reaktionsgemisch, ohne dass gleichzeitig der Katalysator inaktiviert oder zusammen mit der Ameisensäure extrahiert wird.

Alternativ kann auch ein Teil des Reaktionsgemischs abgesondert, mittels des genannten Alkohols extrahiert und die verbleibende, den Katalysator enthaltende Lösung wieder dem restlichen Reaktionsgemisch zugeführt werden.

Der Alkohol hat als Extraktionsmittel den Vorteil, dass er den Katalysator nicht inaktiviert und die Wirkung des Katalysators bei der Erzeugung von Ameisensäure in dem Reaktionsgemisch allenfalls hemmt. Die Extraktion kann somit während der Erzeugung der Ameisensäure in dem Reaktionsgemisch durchgeführt werden.

Grundsätzlich ist die Extraktion von Ameisensäure mittels linearer primärer Alkohole aus dem Stand der Technik bekannt. Dem Fachmann ist jedoch aus Khenkin, A. M. und Neumann, R., J. AM. CHEM. SOC. 2008, 130, 14474 bis 14476 bekannt, dass primäre Alkohole in Gegenwart eines Polyoxometallat-Katalysators durch Oxidation gespalten werden. Die Erfinder der vorliegenden Anmeldung haben jedoch herausgefunden, dass die in Khenkin, A. M. und Neumann, R., J. AM. CHEM. SOC. 2008, 130, 14474 bis 14476 beschriebene Umsetzung primärer Alkohole durch das genannte Polyoxometallat-Ion vermieden werden kann, wenn das Reaktionsgemisch in einem protischen und nicht in einem aprotischen Lösungsmittel vorliegt. Ein linearer primärer Alkohol könnte unter den in der genannten Veröffentlichung beschriebenen Reaktionsbedingungen nicht zur Extraktion der Ameisensäure verwendet werden, weil er dann in Gegenwart des Katalysators unter Spaltung einer C-C-Bindung oxidiert werden würde. Es wurde jedoch erkannt, dass sich lineare primäre Alkohole in protischem Lösungsmittel auch in Gegenwart des genannten Polyoxometallat-Ions zur Extraktion von in dem Reaktionsgemisch gebildeter Ameisensäure eignen.

Bei einer Ausgestaltung der Erfindung weist der lineare primäre Alkohol 6, 7 oder 8 Kohlenstoffatome auf. Es kann sich dabei z. B. um 1-Hexanol oder 1-Heptanol handeln.

Bei einem Ausführungsbeispiel ist das protische Lösungsmittel ein organischer Stoff oder das Reaktionsgemisch umfasst einen organischen Stoff. Dabei kann es sich beispielsweise um ein alpha-Hydroxyaldehyd, eine alpha-Hydroxycarbonsäure, ein Kohlenhydrat oder ein Glycosid handeln.

Bei dem alpha-Hydroxyaldehyd, der alpha-Hydroxycarbonsäure, dem Kohlenhydrat oder dem Glycosid kann es sich um ein Monosaccharid, insbesondere eine Aldose, Disaccharid, Oligosaccharid oder Polysaccharid, Stärke, Zellulose, Hemizellulose, Glucose, Saccharose, Xylose, Zellobiose, Xylan, ein Heterooligosaccharid, ein Heteropolysaccharid, Glycolsäure oder Milchsäure oder einen das alpha-Hydroxyaldehyd, die alpha-Hydroxycarbonsäure, das Kohlenhydrat oder das Glycosid enthaltenden Reststoffen oder, insbesondere nachwachsenden, insbesondere unbehandelten, Rohstoff handeln. Unbehandelt bedeutet dabei, dass dieser zuvor nicht chemisch aufgeschlossen worden ist. Bei dem Reststoff oder dem nachwachsenden Rohstoff kann es sich um eine Pflanze, einen Pilz oder Bakterien oder Bestandteile von Pflanzen, Pilzen oder Bakterien, Holz, insbesondere in Form von Holzmehl oder Holzspänen, Papier, insbesondere Altpapier, Algen, Cyanobakterien oder Silage handeln. Das alpha-Hydroxyaldehyd, die alpha-Hydroxycarbonsäure, das Kohlenhydrat oder das Glycosid kann auch ein Gemisch aus mindestens zwei der genannten Substanzen umfassen oder aus mindestens einer der genannten Substanzen oder dem Gemisch entstanden sein, wie es z. B. bei Torf oder Braunkohle der Fall ist. Viele der genannten Rohstoffe fallen als Nebenprodukte, beispielsweise bei der Papierherstellung oder der Holzverarbeitung an.

Bei einer Ausgestaltung des erfindungsgemäßen Verfahrens ist das protische Lösungsmittel polar. Das bedeutet, dass die Elektronegativitätsdifferenz in einer Bindung der Atome des Lösungsmittels größer als 0,1, insbesondere größer als 0,4 ist. Bei dem protischen Lösungsmittel kann es sich beispielsweise um Wasser handeln.

Nach der Extraktion kann der lineare primäre Alkohol zumindest von einem Teil der Ameisensäure getrennt und dem Reaktionsgemisch zur weiteren Extraktion von Ameisensäure zugeführt werden. Das Trennen von zumindest einem Teil der Ameisensäure kann z. B. durch Destillation oder durch Absondern der Ameisensäure durch Bildung eines Formiats erfolgen. Zur Bildung eines Formiats kann die Ameisensäure mit einem Hydroxid unter Entstehung einer Lösung des Formiats umgesetzt werden. Der lineare primäre Alkohol kann dann durch Verdampfen vom als Feststoff zurückbleibenden Formiat abgesondert und durch anschließende Kondensation als Flüssigkeit zurückgewonnen und optional wieder zur Extraktion verwendet werden.

Nachfolgend wird die Erfindung anhand von Ausführungsbeispielen näher erläutert.

Bei einem ersten Ausführungsbeispiel wird ein Gemisch, welches 0,91 g (0,5 mmol) H₈[PV₅Mo₇O₄₀] und 10,91 g Ameisensäure (FA) in 50 ml Wasser enthält, bei 90°C für eine Stunde unter Rühren mit den aus der folgenden Tabelle 1 ersichtlichen unterschiedlichen Extraktionsmitteln extrahiert. Zur Ermittlung der jeweiligen Konzentration der Ameisensäure in der organischen und in der wässrigen Phase wurde der Ansatz danach in einen Scheidetrichter überführt, in welchem sich beide Phasen trennten. Proben beider Phasen wurden dann mit Hilfe von ¹H-NMR analysiert, um die Ameisensäurekonzentration zu bestimmen. Als externer Standard diente dabei Benzol.

**Tabelle 1:**

| Extraktionsmittel | Verteilungskoeffizient Kc_{FA, org.}/c_{FA, aqu.} | Selektivität K_{FA}/K_{Wasser} |
|---|---|---|
| 1-Hexanol | 0.94 | 8.6 |
| 1-Heptanol | 0.67 | 4.4 |
| Di-n-butylether | 0.22 | 1.2 |
| Di-isopropylether | 0.40 | 2.5 |
| Butylethylether | 0.49 | 2.7 |
| Benzylformiat | 0.46 | 2.6 |
| Heptylformiat | 0.42 | 2.2 |

Aus der obigen Tabelle 1 ist ersichtlich, dass die Extraktionsmittel 1-Hexanol und 1-Heptanol besonders gut zur Extraktion geeignet sind.

Der Verteilungskoeffizient C_{FA, org.}/C_{FA, aqu.} gibt das Verhältnis der Konzentration der Ameisensäure in der organischen Phase des jeweiligen Extraktionsmittels (C_{FA, org.}) zur Konzentration der Ameisensäure in der wässrigen Phase (C_{FA, aqu.}) an. Je größer der Verteilungskoeffizient bei dem jeweiligen Extraktionsmittel ist, desto höher ist die Kapazität des jeweiligen Extraktionsmittels für die Aufnahme von Ameisensäure und desto besser ist das Extraktionsmittel zur Extraktion von Ameisensäure aus der wässrigen Phase geeignet. Die Selektivität K_{FA}/K_{Wasser} gibt das Verhältnis der Verteilungskoeffizienten von Ameisensäure und Wasser an. Je höher diese Selektivität ist, desto größer ist der Anteil des Katalysators und des Substrats, welcher in der wässrigen Phase verbleibt. Ein Extraktionsmittel ist also umso besser geeignet, je höher der Verteilungskoeffizient und je höher die Selektivität des jeweiligen Extraktionsmittels ist.

In einem weiteren Ausführungsbeispiel wurde untersucht, ob das Extraktionsmittel während der Oxidation eines organischen Stoffs zur Erzeugung von Ameisensäure dem Reaktionsgemisch zugesetzt werden kann, um eine in-situ-Extraktion von Ameisensäure während der Oxidationsreaktion zu erreichen. Das Reaktionsgemisch enthielt dabei 4,5 g Glucose, 1,82 g (1 mmol) H₈[PV₅Mo₇O₄₀] gelöst in 100 ml Wasser sowie 100 g des jeweiligen Extraktionsmittels. Die Reaktion erfolgte bei 90°C und 20 bar Sauerstoffpartialdruck bei unterschiedlichen Rührgeschwindigkeiten für 6 Stunden. Bei der Reaktion wird die Glucose zu Ameisensäure und CO₂ umgesetzt. Die Gesamtkohlenstoffausbeute in Form von Ameisensäure und CO₂ bezogen auf die ursprünglich eingesetzte Menge an Kohlenstoff und das Verhältnis der erzeugten molaren Mengen an Ameisensäure zur erzeugten molaren Menge an CO₂ wurde mittels Gaschromatographie und ¹H-NMR unter Verwendung von Benzol als externem Standard bestimmt. Bei der Gesamtkohlenstoffausbeute sind Mol-% bezogen auf die insgesamt eingesetzten Mol Kohlenstoff angegeben.

**Tabelle 2:**

| Rührgeschwindigkeit (Umdrehungen/min) | Extraktionsmittel | Gesamtkohlenstoffausbeute FA+CO₂ (Mol-% bezogen auf eingesetzte Mol C) | Selektivität FA[Mol]:CO₂ [Mol] | Verteilungskoefficient c_{FA, org.}/c_{FA, aqu.} |
|---|---|---|---|---|
| 600 | 1-Hexanol | 25 | 88:12 | 0.88 |
| 600 | 1-Heptanol | 59 | 89:11 | 0.59 |
| 800 | 1-Hexanol | 16 | 80:20 | 0.69 |
| 800 | 1-Heptanol | 44 | 81:19 | 0.54 |
| 1000 | 1-Hexanol | 24 | 80:20 | 0.65 |
| 1000 | 1-Heptanol | 40 | 82:18 | 0.55 |
| 1200 | 1-Hexanol | 22 | 80:20 | 0.97 |
| 1200 | 1-Heptanol | 37 | 78:22 | 0.60 |
| 1400 | 1-Hexanol | 27 | 86:14 | 0.77 |
| 1400 | 1-Heptanol | 43 | 82:18 | 0.67 |
| 1000 | kein Extraktionsmittel | 100 | 53:47 | - |

Die in der obigen Tabelle 2 dargestellten Ergebnisse zeigen, dass die Gegenwart des Extraktionsmittels bei den entstehenden Reaktionsprodukten zu einem starken Anstieg der Selektivität für Ameisensäure führte, d. h. von den bei der Reaktion entstehenden Reaktionsprodukten Ameisensäure und CO₂ ist der Anteil an Ameisensäure an den Reaktionsprodukten stark gesteigert. Dieser Anstieg an Selektivität geht jedoch unabhängig von der Rührgeschwindigkeit mit einer geringeren Umsetzungsrate einher, d. h. die Oxidation der Glucose erfolgt in dem das Extraktionsmittel enthaltendem System langsamer als in einem System ohne das Extraktionsmittel. Bei weiteren Experimenten wurde herausgefunden, dass die erhöhte Selektivität keine Folge der langsameren Glucoseoxidation ist, sondern eine Folge der sehr effektiven Ameisensäureextraktion in dem biphasischen Reaktionssystem.

Das Experiment zeigt, dass die durch den Katalysator bewirkte Umsetzung eines organischen Stoffs zu Ameisensäure durch das Extraktionsmittel zwar gehemmt ist, diese Hemmung jedoch zumindest teilweise durch eine höhere Selektivität zugunsten der Ameisensäure ausgeglichen wird.

## Patentansprüche

1. Verfahren zum Absondern von Ameisensäure aus einem Reaktionsgemisch, welches neben der Ameisensäure ein Polyoxometallat-Ion der allgemeinen Formel [PMoₓV_{y}O₄₀]ⁿ⁻ umfasst, wobei 6 ≤ x ≤ 11, 1 ≤ y ≤ 6, x + y = 12 und 3 < n < 10, wobei n, x und y jeweils eine ganze Zahl ist, wobei das Absondern durch eine Extraktion mittels eines linearen primären Alkohols erfolgt, wobei die Kohlenstoffkette des Alkohols jeweils 5 bis 12 Kohlenstoffatome aufweist, wobei das Reaktionsgemisch in einem protischen Lösungsmittel vorliegt.

2. Verfahren nach Anspruch 1, wobei der lineare primäre Alkohol 6, 7 oder 8 Kohlenstoffatome aufweist.

3. Verfahren nach einem der vorhergehenden Ansprüche, wobei der lineare primäre Alkohol 1-Hexanol oder 1-Heptanol umfasst.

4. Verfahren nach einem der vorhergehenden Ansprüche, wobei das protische Lösungsmittel ein organischer Stoff ist oder das Reaktionsgemisch einen organischen Stoff umfasst.

5. Verfahren nach Anspruch 4, wobei es sich bei dem organischen Stoff um ein alpha-Hydroxyaldehyd, eine alpha-Hydroxycarbonsäure, ein Kohlenhydrat oder ein Glycosid handelt.

6. Verfahren nach einem der vorhergehenden Ansprüche, wobei das protische Lösungsmittel polar ist.

7. Verfahren nach einem der vorhergehenden Ansprüche, wobei das protische Lösungsmittel Wasser ist.

8. Verfahren nach einem der vorhergehenden Ansprüche, wobei der lineare primäre Alkohol nach der Extraktion zumindest von einem Teil der Ameisensäure getrennt und dem Reaktionsgemisch zur weiteren Extraktion von Ameisensäure zugeführt wird.

9. Verfahren nach einem der vorhergehenden Ansprüche, wobei n = 3 + y.

10. Verfahren nach einem der vorhergehenden Ansprüche, wobei das Absondern durch die Extraktion während einer Reaktion zur Erzeugung der Ameisensäure erfolgt.

11. Verfahren nach Anspruch 10, wobei das Absondern bei einer Temperatur von 50°C bis 120°C erfolgt.

## Claims

1. A method for separating formic acid from a reaction mixture comprising in addition to formic acid a polyoxometallate ion of the general formula [PMoₓV_{y}O₄₀]ⁿ⁻, where 6 ≤ x ≤ 11, 1 ≤ y ≤ 6, x + y = 12 and 3 < n < 10, where n, x and y are each integers, the separation being accomplished by extraction by means of a linear primary alcohol, wherein the carbon chain of the alcohol has five to twelve carbon atoms, and the reaction mixture is in a protic solvent.

2. The method according to claim 1, wherein the linear primary alcohol has six, seven or eight carbon atoms.

3. The method according to any one of the preceding claims, wherein the linear primary alcohol comprises 1-hexanol or 1-heptanol.

4. The method according to any one of the preceding claims, wherein the protic solvent is an organic substance or the reaction mixture comprises an organic substance.

5. The method according to claim 4, wherein the organic substance is an α-hydroxyaldehyde, an α-hydroxycarboxylic acid, a carbohydrate or a glycoside.

6. The method according to any one of the preceding claims, wherein the protic solvent is polar.

7. The method according to any one of the preceding claims, wherein the protic solvent is water.

8. The method according to any one of the preceding claims, wherein the linear primary alcohol is separated from at least a portion of the formic acid after extraction and added to the reaction mixture for further extraction of formic acid.

9. The method according to any one of the preceding claims, wherein n = 3 + y.

10. The method according to any one of the preceding claims, wherein the separation takes place by extraction during a reaction to produce formic acid.

11. The method according to claim 10, wherein the separation takes place at a temperature of 50°C to 120°C.

## Revendications

1. Procédé de séparation d'acide formique d'un mélange réactionnel qui comprend outre l'acide formique un ion polyoxométallate de la formule générale [PMoₓV_{y}O₄₀]ⁿ⁻, dans laquelle 6 ≤ x ≤ 11, 1 ≤ y ≤ 6, x + y = 12 et 3 < n < 10, dans laquelle n, x et y sont chacun un nombre entier, dans lequel la séparation s'effectue par une extraction au moyen d'un alcool primaire linéaire, dans lequel la chaîne des carbones de l'alcool présente à chaque fois 5 à 12 atomes de carbone, dans lequel le mélange réactionnel est présent dans un solvant protique.

2. Procédé selon la revendication 1, dans lequel l'alcool primaire linéaire présente 6, 7 ou 8 atomes de carbone.

3. Procédé selon une des revendications précédentes, dans lequel l'alcool primaire linéaire comprend le 1-hexanol ou 1-heptanol.

4. Procédé selon une des revendications précédentes, dans lequel le solvant protique est une substance organique ou le mélange réactionnel comprend une substance organique.

5. Procédé selon la revendication 4, dans lequel il s'agit pour la substance organique d'un alpha-hydroxyaldéhyde, d'un acide alpha-hydroxycarboxylique, d'un hydrate de carbone ou d'un glycoside.

6. Procédé selon une des revendications précédentes, dans lequel le solvant protique est polaire.

7. Procédé selon une des revendications précédentes, dans lequel le solvant protique est de l'eau.

8. Procédé selon une des revendications précédentes, dans lequel l'alcool primaire linéaire est séparé après l'extraction d'au moins une partie de l'acide formique et alimenté au mélange réactionnel pour l'extraction supplémentaire d'acide formique.

9. Procédé selon une des revendications précédentes, dans lequel n = 3 + y.

10. Procédé selon une des revendications précédentes, dans lequel la séparation s'effectue par l'extraction pendant une réaction pour la génération de l'acide formique.

11. Procédé selon la revendication 10, dans lequel la séparation s'effectue à une température de 50°C à 120°C.
